# EUROPEAN PATENT APPLICATION

(11) **EP 4 231 310 A1**
(43) Date of publication of application: **23.08.2023**
(21) Application number: 22157074.0
(22) Date of filing: 16.02.2022
(51) Int. Cl.: G16H 30/20

(54) **SYSTEMS AND METHODS FOR TRAINING AND APPLICATION OF MACHINE LEARNING ALGORITHMS FOR MICROSCOPE IMAGES**

(71) Applicant: Leica Instruments (Singapore) Pte Ltd, Singapore 608924 (SG)
(72) Inventor: ORMAZ, Milo, 8706 Meilen (CH); SCHWEIZER, Jochen, 86938 Schondorf am Ammersee (DE); THEMELIS, George, 88131 Lindau (DE)
(74) Representative: DehnsGermany Partnerschaft von Patentanwälten

(57) **Abstract**

The invention essentially relates to a system (150) comprising one or more processors (152) and one or more storage devices (154), for training of a machine-learning algorithm (160), wherein the system (150) is configured to: receive training data (142), the training data comprising: images (120) showing tissue; adjust the machine-learning algorithm (160) based on the training data (142), such that the machine-learning algorithm generates instruction data for at least part of said tissue shown in said images (120), wherein said instruction data is indicative of an action to be performed on said tissue; and provide the trained machine learning algorithm (164). The invention also relates to a system for applying such machine learning algorithm and to corresponding methods.

## Description

### Technical Field

The present invention essentially relates to a system and a method for training of a machine-learning algorithm, based on images, e.g. fluorescence images from a surgical microscope, to a trained machine-learning algorithm, and to a system and a method for the application of such machine-learning algorithm.

### Background

In surgical microscopy, e.g., for tumour surgeries or the like, a surgeon can view the surgical site or the patient by using the surgical microscope. A decision which tissue or sections thereof shall be resected or removed, e.g., tumour tissue, typically has to be made by the surgeon.

### Summary

In view of the situation described above, there is a need for improvement in providing assistance as to which tissue sections to remove or not to remove. According to embodiments of the invention, a system and a method for training of a machine-learning algorithm, based on images, e.g., from a surgical microscope, a trained machine-learning algorithm, and a system and a method for the application of such machine-learning algorithm with the features of the independent claims are proposed. Advantageous further developments form the subject matter of the dependent claims and of the subsequent description.

An embodiment of the invention relates to a system comprising one or more processors and one or more storage devices, for training of a machine-learning algorithm, e.g., an artificial neural network. The system is configured to: receive training data, wherein the training data comprises: images showing tissue. Such images can be acquired by means of a surgical microscope or from radiology or in another way. The system is further configured to determine an adjusted (trained) machine-learning algorithm based on the training data, i.e., the machine-learning algorithm is trained, such that the machine-learning algorithm generates instruction data for at least part of said tissue shown in said images, wherein said instruction data is indicative of an action to be performed on said tissue, e.g., during a surgery. Such instruction data can be or include a resection boundary or the like. The system is further configured to provide the trained machine-learning algorithm, which is then, e.g., configured to provide such instruction data during the intraoperative use of a surgical microscope.

In surgeries, tumour and other harmful tissue can be resected or removed from a patient or a patient's brain, non-harmful tissue or the like. As mentioned before, the surgeon has to decide (during the surgery), which tissue or sections thereof exactly to resect or not to resect. In particular in neurosurgical oncology, there is an additional limitation related to the side-effects of neural tissue excision, as the removal of vital brain tissue, even if the tissue is cancerous, might lead to brain function compromise, which might be more severe than the damage caused by the tumor. Thus, in neurosurgical oncology, a successful surgical intervention is not always the complete excision of diseased (harmful) tissue, but rather a fine balance between tumor excision and neural tissue preservation is preferred. Also, it has turned out that sometimes tumour is infiltrative and scattered outside a solid tumour component (core); such tumour might be difficult to detect during surgery.

Typically, the planning of an optimal surgical intervention is done empirically, relying on the surgeon's experience to evaluate all preoperative and intraoperative data. These data include, e.g., MRI tumour delineation, intraoperative visual assessment (white light imaging), intraoperative tumour delineation (based on, e.g., fluorescence imaging), and preoperative and intraoperative functional areas determination. The collective evaluation of all data is quite a challenging task for the surgeon, especially during the surgical operation. With the system for training of a machine-learning algorithm mentioned above, a way is provided to indicate - by means of the instruction data - to a surgeon, which tissue or sections of tissue to resect or not to resect, automatedly by such machine-learning algorithm. The training data used for training can, e.g., be collected from many surgeries or other situations or cases, as will be mentioned later.

According to a further embodiment of the invention, the training data further comprises patient data, which includes information about patients correlated to said tissue shown said images. Said patient data, preferably, comprises at least one of the following parameters for said patients: age, gender, type and dosage of oncological treatment, remaining life time, blood pressure, cholesterol, tumour recurrence, loss of at least part of one or more cognitive functions, and other measurable data in patient's health file. This allows supervised training or learning of the machine-learning algorithm considering different features and measures leading to different decisions of whether to resect harmful tissue or not.

According to a further embodiment of the invention, the machine-learning algorithm is adjusted such that the machine-learning algorithm generates said instruction data such that the instruction data is based at least in part on at least parts of said patient data. Said patient data is to be provided to the trained machine-learning algorithm as input data for its application. In this way, the instructions provided to the surgeon are individual for the current patient Depending on the number of different kinds of patient data to be used, the instructions will be more or less individualized.

According to a further embodiment of the invention, the training data further comprises annotations on the images. Said annotations are indicative of at least one of: classes or types of tissue shown in said images, and actions to be performed or having been performed on said tissue.

Such annotations indicative of classes or types of sections of said tissue shown in the corresponding images can be labels like "tumour or not tumour" (i.e., classes like "tumour", "not tumour"), e.g., received from histopathology and/or other processes after resection. Annotations indicative of actions to be performed or having been performed on said tissue can be labels like "do not resect despite being tumour", "do resect only part of said section" or the like. According to a further embodiment, the machine-learning algorithm is based on classification. This allows training for a classification type of machine-learning algorithm, in particular based on at least part of said annotations. Such training requires said annotations on the images as training data. With, in addition, said patient data and said actions to be performed or having been performed as further training data, such a classification type of machine-learning algorithm, after training, allows determining, in a real-time imaging, whether specific sections of tissue should be resected or not. Note that a classification type of machine-learning algorithm does not require calculation of the remaining lifetime of the patient after surgery, because it classifies tissue according to the "correlation" of different signals of images with the provided annotation. Such signals in images can comprise, for example, the pixel's colour, spectrum, fluorescence intensity, glossiness and the like. A learned correlation can then be used to predict classes of different brain tissues, for example.

According to a further embodiment of the invention, said machine learning algorithm is based on regression. This allows training for a regression type of machine-learning algorithm. That regression can, preferably, be based on at least part of said annotations on the images. In other words, another (or additional) approach is a regression type machine learning algorithm, which can be trained by finding "corretation(s)" between images, features in images like pixel's colour, spectrum, fluorescence intensity, glossiness and the like, and a remaining life time (life duration) after surgery. Such a regression type of machine-learning algorithm, thus, after training, allows determining, in a real-time imaging, whether specific sections of tissue should be resected or not; this holds true in particular for a single calculated life expectancy after surgery. Note that multiple calculated life expectancies are preferably based on multiple proposed resection boundaries from which one is selected.

A differences between regression and classification are, in particular, that regression looks at the outcome of the whole surgery, while classification simply classifies different areas of tissue individually and that regression, at least theoretically, does not need annotations

According to a further embodiment of the present invention, said trained machine-learning algorithm is determined based on unsupervised learning, i.e., the training is unsupervised training or learning. Such training method does not require desired output data (annotations or actions to be performed) as training data but only said images, e.g., from a surgical microscope obtained during a surgery.

According to a further embodiment of the present invention, the training data further comprises at least one of: radiology images or scans, ultrasound images, endoscope images, neuromonitoring information. Each of these can, preferably, correspond to said images showing tissue, with respect to field of view, for example. Such radiology images or scans can result from or be obtained by, e.g., Magnetic Resonance Imaging (MRI), Computer Tomography (CT) or the like. This further improves the training of the machine-learning algorithm by providing additional information within the training data. For example, specific structures, in particular inside the tissue, which might indicate harmful or non-harmful tissue or tissue sections cannot such clearly be seen in (microscope) images. In particular, said radiology images are obtained from radiology scans and have the same or a similar field of view as the corresponding (microscope) images have. These radiology images and the other kind of images and information mentioned before also can be annotated, preferably in the same way as mentioned for the images in general; this allows increasing the number of images and information and increasing type of information to be used as training data.

According to a further embodiment of the present invention, said instruction data comprises a boundary, which is indicative of an area of said tissue, in which area said action is to be performed on said issue. In this way, the instruction data may say that within a specific boundary, all tissue is to be resected. The surgeon then will do so. This increases confidence as to decisions what to resect or not to resect provided to and made by the surgeon.

According to a further embodiment of the present invention, said boundary is determined out of multiple boundaries with different values for a parameter relating to said tissue or a patient from which said tissue is from. Then, said boundary is determined such that said value for said parameter is maximised. This is particularly the case for the regression type of machine-learning algorithm. For example, said parameter can be remaining life time of said patient after the current surgery. Then, the boundary to be determined out of the multiple ones is the one which corresponds to the longest remaining life time. Another (or additional) parameter might be life quality after surgery, e.g., indicate of whether the patient still can use all his neurological skills or the like. Note that this might mean that not all harmful tissue is within said boundary.

According to a further embodiment of the present invention, said instruction data comprises different indicators for different actions to be performed on said tissue. Such different indicators can be, for example, different colours and/or shadings. This is particularly the case for the classification type of machine-learning algorithm. It allows the surgeon to see what type of tumour is present, for example, and what to do.

According to a further embodiment of the present invention, the machine-learning algorithm is adjusted such that the machine-learning algorithm generates said instruction data being overlaid to said microscope images. For example, this may result in the real-time image overlaid by a boundary, directly indicating which tissue to resect or not to resect.

A further embodiment of the invention relates to a computer-implemented method for training of a machine-learning algorithm. The method comprises receiving training data, wherein the training data comprises images showing tissue. The method further comprises adjusting the machine-learning algorithm based on the training data, such that the machine-learning algorithm generates instruction data for at least part of said tissue shown in said images; said instruction data is indicative of an action to be performed on said tissue. The training data can further comprise said patient data and/or said other types of images mentioned. The method also comprises providing the trained machine-learning algorithm, e.g., to a user or a system for application.

A further embodiment of the invention relates to a trained machine-learning algorithm, which is trained by receiving training data, wherein the training data comprises images showing tissue; and adjusting the machine learning algorithm based on the training data, such that the machine-learning algorithm generates instruction data for at least part of said tissue shown in said images; said instruction data is indicative of an action to be performed on said tissue. The training data can further comprise said patient data and/or said other types of images mentioned.

A further embodiment of the invention relates to a system comprising one or more processors and one or more storage devices, for providing instruction data, e.g., in the form of a boundary or encirclement boundary, for tissue shown in a microscope image, for example, during a surgery. This system is configured to receive input data, wherein the input data comprises a microscope image from a surgical microscope obtained during a surgery. Said microscope image shows tissue of a patient. The input data can further comprise pre-operative radiology images or scans and/or others of said other types of images mentioned. The input data can further comprise patient data like relevant data from the patient's health record. The system is further configured to generate instruction data for at least part of said tissue shown in said microscope image, by applying a machine-learning algorithm. Said instruction data is indicative of an action to be performed on said tissue. The system is further configured to provide output data, which comprises the instruction data for said microscope image. For example, said instruction data (e.g., a boundary or resection boundary) might be overlaid onto said microscope image.

According to a further embodiment of the present invention, the input further comprises patient data. The instruction data then is determined based at least in part on at least parts of said patient data. Such patient data might be provided to the system prior to starting the surgery.

A further embodiment of the invention relates to a surgical microscopy system, which comprises a surgical microscope, an image sensor and the system for providing instruction data, according to the above-mentioned embodiment

A further embodiment of the invention relates to a computer-implemented method for providing instruction data for tissue shown in a microscope image. This method comprises receiving input data, which comprises a microscope image from a surgical microscope obtained during a surgery; the microscope image shows tissue of a patient. The method further comprises generating instruction data for at least part of said tissue shown in said microscope image, by applying a machine-learning algorithm; said instruction data is indicative of an action to be performed on said tissue. The input data can further comprise pre-operative radiology images or scans and/or others of said other types of images mentioned. The input data can further comprise patient data like relevant data from the patient's health record. The method further comprises providing output data, which comprises the instruction data for said microscope image.

A further embodiment of the invention relates to method for providing an image and instruction data to a user, e.g., a surgeon, using a surgical microscope. This method comprises illuminating tissue of a patient, capturing a microscope image of the tissue, which microscope image shows the tissue. The method further comprises generating instruction data for at least part of said tissue shown in said microscope image by applying a machine-learning algorithm; said instruction data is indicative of an action to be performed on said tissue. The method further comprises providing said microscope image and said instruction data to the user of the surgical microscope. The method can further comprise importing data pre-operative radiology images or scans and/or others of said other types of images mentioned, and/or patient data like relevant data from the patient's health record.

With respect to advantages and further embodiments of the methods, it is referred to the remarks of the systems, which apply here correspondingly.

A further embodiment of the invention relates to a computer program with a program code for performing the method of above, when the computer program is run on a processor.

Further advantages and embodiments of the invention will become apparent from the description and the appended figures.

It should be noted that the previously mentioned features and the features to be further described in the following are usable not only in the respectively indicated combination, but also in further combinations or taken alone, without departing from the scope of the present invention.

### Short description of the Figures

- Fig. 1: schematically shows a system for training of a machine-learning algorithm according to an embodiment of the invention;
- Fig. 2: schematically shows a system for providing instruction data according to a further embodiment of the invention;
- Fig. 3: schematically shows a way of how to create annotations for use in training of a machine-learning algorithm according to an embodiment of the invention;
- Fig. 4a: schematically shows instruction data generated by application of a machinelearning algorithm according to an embodiment of the invention;
- Fig. 4b: schematically shows instruction data generated by application of a machinelearning algorithm according to an embodiment of the invention;
- Fig. 5: schematically shows a method for training of a machine-learning algorithm according to an embodiment of the invention;
- Fig. 6: schematically shows a method for correcting a microscope image according to a further embodiment of the invention; and
- Fig. 7: schematically shows a method for providing an image to a user according to a further embodiment of the invention.

### Detailed Description

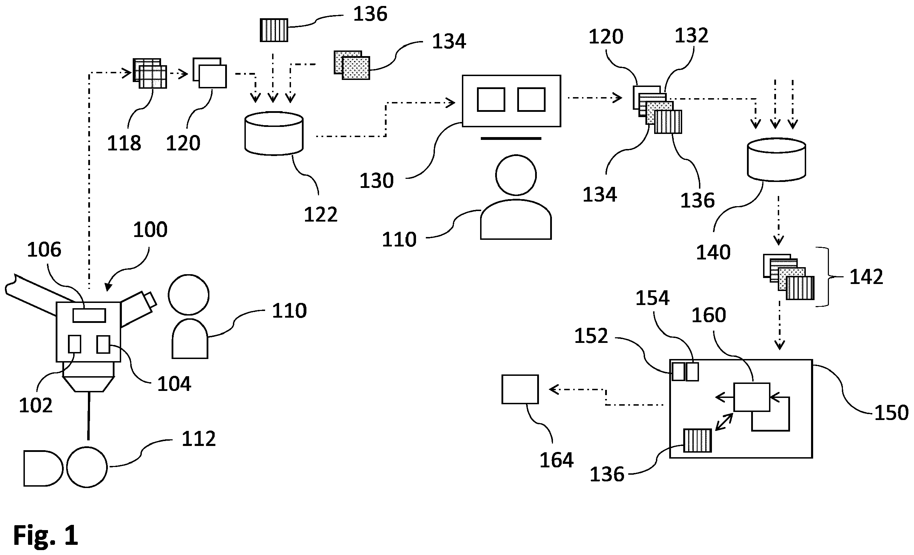

Fig. 1 schematically illustrates a system 150 for training of a machine-learning algorithm according to an embodiment of the invention. The system 150 comprises one or more processors 152 and one or more storage devices 154. For example, system 150 can be a computer or other server system.

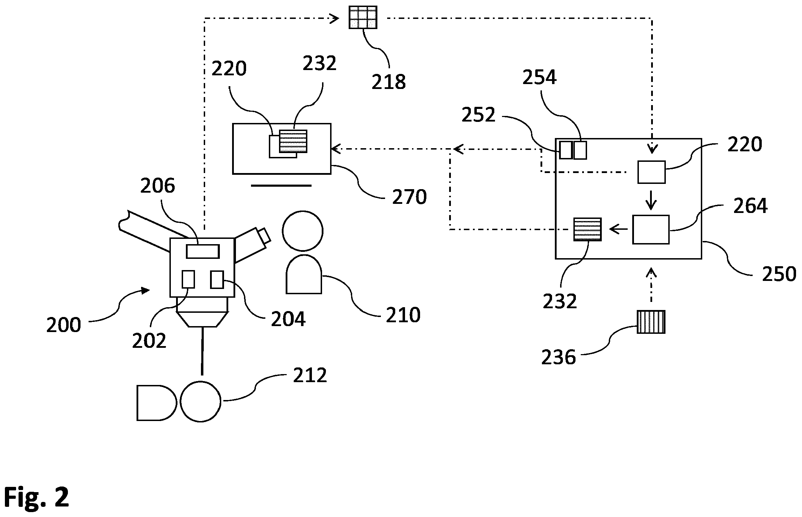

System 150 is configured to receive training data 142; such training data 142 comprises images like microscope images 120 from a surgical microscope 100 obtained during a surgery. The (microscope) images show tissue like a brain of a patient, including tumour and/or other harmful tissue. System 150 is further configured to adjust the machine-learning algorithm 160 based on the training data 142, such that the machine-learning algorithm generates instruction data for at least part of said tissue shown in said images 120, when the machine-learning algorithm is applied; this will be explained in more detail with respect to Fig. 2. System 150 is further configured to provide the adjusted - or trained - machine learning algorithm 164, e.g., for eventual application.

According to a further embodiment of the invention, the training data 142 comprises annotations 132 on the images like the microscope images 120. Such annotations can include or be labels indicating, whether a section in the microscope image 120 is tumour or not, for example. Such label can also indicate, whether a section should be resected or not In this way, the annotations can be indicative of classes of sections of said tissue shown in the corresponding microscope images 120 and of actions to be performed or having been performed on said tissue; such annotations can be used for a machine-learning algorithm of at least one of the classification and regression types.

According to a further embodiment of the invention, the training data 142 comprises patient data 136, which includes information about patients correlated to said tissue shown said images 120. Said patient data 136, according to an embodiment of the invention, comprises at least one of the following parameters for said patients: age, gender, type and dosage of oncological treatment, remaining life time (after surgery), blood pressure, cholesterol etc. (i.e. all measurable data in patient's health file, as well as data describing patient's life quality after surgery such as tumour recurrence and loss of some cognitive capabilities (e.g. speech), which could both be categorical variables). During statistical analysis, some variables (e.g. gender) might be found as redundant or irrelevant

According to a further embodiment of the invention, the training data 142 comprises radiology images 134, which may be obtained from radiology scans, in particular, by finding the same field of view in the radiology scan, which corresponds to said microscope images 120. These radiology images 134 can be annotated like the microscope images 120. Also, ultrasound images, endoscope images, and neuromonitoring information can be comprised by the training data 142.

In the following, it will be explained in more detail how to obtain said microscope images 120, said annotations 132, and said patient data 136, which can be used as training data 142, referring to Fig. 1

During a surgery, a surgeon 110 (user) uses a surgical microscope 100 in order to view the surgical site, e.g., a patient 112 or a patient's brain. Said surgical microscope 100 can comprise an illumination optics 102 for visible light and an illumination optics 104 for excitation light for exciting fluorophores in tissue of the patient 112. Alternatively, appropriate filters for filtering wavelengths of light required for excitation and emission from tissue might be used. An image sensor 106, e.g., a detector or camera, acquires fluorescence light, emanating from the illuminated tissue. Image sensor 106 might also acquire visible light. Alternatively, another image sensor can be used for visible light. In this way, raw data 118 for microscope images 120 - in particular visible light images and/or fluorescence light images, or combined visible light and fluorescence light images - is produced. Such raw data can be processed in order to obtain the (final) microscope images 120. Such processing of raw data can take place inside the image sensor 106 or another processor included in the surgical microscope 100 or in a further external processor (not shown here). Said processing of raw data 118 can include, for example, applying filters or the like. Said microscope images 120 are then stored in a database 122.

It is noted that such microscope images 120 can be produced or acquired during the surgery several times, in particular, before, during and after resection of harmful tissue like tumour. In this way, multiple microscope images 120 can be acquired from a single surgery. In the same way, further microscope images 120 can be acquired before, during and after other (different) surgeries, which are in particular of the same type, i.e., which include resection of the same kind or type of harmful tissue. This allows collecting and storing a huge amount of microscope images 120 in the database 122. A further way to increase the amount of images and variety of information is by obtaining radiology images 134 from radiology scans as mentioned above, ultrasound images, and endoscope images. Also, information from neuromonitoring can be acquired. These can also be stored in the database 122.

These microscope images 120 and radiology images 134 can then be viewed, e.g., on a computing system with a display running an annotation application 130. The surgeon 110 or any other competent user can view pairs of microscope images of the same surgery, from the microscope images 120 in the database 122, in said annotation application 130. Such pairs of microscope images comprise an image prior to resection, showing the marked sections of tissue, and an image after resection.

It is noted that, preferably, such microscope images can comprise marked sections. Such (harmful) sections of tissue can be marked by means of fluorophores or other staining material or markers, typically given to the patient A typical fluorophore for marking harmful tissue like tumour is, but not limited to, 5-ALA (5-aminolevulinic acid). There are also other fluorophores that might be used. During the surgery, the surgical site is then illuminated with excitation light of appropriate wavelength for exciting the fluorophore or marker to emit fluorescence light. This emitted light can be captured or acquired by the surgical microscope or an imager thereof and, e.g., be displayed on a display. Fluorescence light images can be used here; the imaging method is also called fluorescence imaging. In this way, the surgeon can more easily identify tissue, which might have to be resected, or whether tissue, which might have to be resected is still present after resection.

Such marking can help the surgeon or the user of the application 130 to (more easily) identify harmful and non-harmful tissue. Note, however, that such marking is not necessary in order to create annotations.

The image after resection still might show harmful tissue, e.g., marked sections of tissue; these sections typically comprise non-harmful tissue that was (marked but) not considered to be resected during the surgery, or purposely left harmful tissue, which could not have been resected for various reasons (e.g. brain area in charge of some cognitive process such as speech).

For each of such pairs of microscope images 120, annotations 132 can be created, which annotations are indicative of classes of sections of said tissue shown in the corresponding microscope images, like "tumour" or "not tumour"; in addition, such annotations 132 can be indicative of actions to be performed or having been performed on said tissue. For example, such annotation can say "tumour but not to be resected" (for example, if the section of tissue is a brain area in charge of some cognitive process such as speech).

In the same or a similar way, pairs of radiology images 134 and other images can be viewed and annotated. Then, said annotations 132 can also include annotations on radiology images or other images.

These annotations 132 and the patient data 136, preferably together with the microscope images 120 and/or the radiology images 134 and/or other images, can then be stored in a further database 140. It is noted that further microscope images 120, annotations 132 and/or patient data 136, can be produced or created at other places and/or by other persons; these can also be stored in database 140.

Said radiology images or scans 134 can be obtained by means of MRI, CT or the like. Since such radiology images or scans typically provide other details of the tissue than the microscope images do, this improves the training by adding further information.

When the machine-learning algorithm 160 is to be trained, said training data 142, preferably comprising microscope images 120, annotations 132 and/or patient data 136, and radiology images or scans 134 and/or other images or information, can be provided to system 150 from the database 140.

In particular for a machine-learning algorithm of the classification type, in this way, the training data 142 can comprise the microscope images 120 (and radiology images 134) and/or the patient data 136 as input data and the annotations 132 or parts thereof as desired output data of the machine-learning algorithm. The machine-learning algorithm shall provide or generate, for a microscope image 120 of tissue, received as its input, instruction data that is indicative of an action to be performed on said tissue. Such instruction data can, in particular, comprise different colours or other indicators for different actions to be performed (like: to be resected, not to be resected)

In order to obtain this, e.g., weights of the machine-learning algorithm - it may be an artificial neural network - are to be adjusted. For example, such weights might be adjusted iteratively until instruction data for a microscope image 120, created by the machine-learning algorithm, comprises the desired actions based on said annotations.

In particular for a machine-learning algorithm of the regression type, in this way, the training data 142 can comprise the microscope images 120 (and radiology images 134) and/or the patient data 136 as input data of the machine-learning algorithm. The machine-learning algorithm shall provide or generate, for a microscope image 120 of tissue, received as its input, instruction data that is indicative of an action to be performed on said tissue. Such instruction data can, in particular, comprise a boundary for an area, in which said action is to be performed (like: to be resected). Such boundary might be such that if tissue within it is resected, the remaining life time of the patient is maximised.

In order to obtain this, e.g., weights of the machine-learning algorithm - it may be an artificial neural network - are to be adjusted. For example, such weights might be adjusted iteratively until input data or features thereof are correlated to a remaining life time of a patient, from which a microscope image 120 originates.

Eventually, the trained machine-learning algorithm 164 can be provided for further application, e.g., during a surgery.

Fig. 2 schematically illustrates a system 250 for providing instruction data for tissue shown in a microscope image according to a further embodiment of the invention, in particular for application of a (trained) machine-learning algorithm. Fig. 2 also illustrates a surgical microscope 200. The system 250 comprises one or more processors 252 and one or more storage devices 254. For example, system 250 can be a computer or controller; in particular, system 250 can be part of, or integrated into, a surgical microscope, like surgical microscope 200.

Said surgical microscope 200 can comprise an illumination optics 202 for visible light and an illumination optics 204 for excitation light for exciting fluorophores in tissue of a patient 212. Alternatively, appropriate filters for filtering wavelengths of light required for excitation might be used. An image sensor 206, e.g., a detector or camera, acquires fluorescence light, emanating from the illuminated tissue. Image sensor 206 might also acquire visible light. Alternatively, another image sensor can be used for visible light In this way, raw data 218 for a microscope image 220 - in particular visible light images and/or fluorescence light images, or combined visible light and fluorescence light images - is produced. Such raw data can be processed in order to obtain the (final) microscope image 120. Such processing of raw data can take place inside the image sensor 206 or another processor included in the surgical microscope 200 or in a further external processor (not shown here). Said processing of raw data 218 can include, for example, applying filters or the like. Note, that surgical microscope 200 can correspond to surgical microscope 100 of Fig. 1.

During a surgery, a surgeon 210 (user) uses said surgical microscope 200 in order to view the surgical site, e.g., a patient 212 or a patient's brain. By means of surgical microscope 200, a microscope image 220 is acquired. Note, that such microscope images 220 can be acquired sequentially in real-time; in the following, the application of a trained machine-learning algorithm 264 to a single microscope image 220 will be explained. However, it can correspondingly be applied to a sequence of images or to each image (frame) of a video.

System 250 is configured to receive input data, which comprises a microscope image 220 from said surgical microscope 200; said microscope image 220 is obtained or acquired during a surgery and shows tissue of patient 212. Said microscope image 220 might be received from the surgical microscope 200 or its image sensor 206 directly or indirectly; in particular, the image sensor 206 might produce raw data 218, which is then to be processed into final microscope image 220. Said input data further comprises patient data 236, which includes information patient 212 currently undergoing the surgery.

System 250 is further configured to generate instruction data 232 for at least part of said tissue shown in said microscope image 220, by applying a machine-learning algorithm 264. Said machine-learning algorithm 264 preferably corresponds to or is the machine-learning algorithm 164 trained with system 150 described with respect to Fig. 1. Said instruction data 232 is indicative of an action to be performed on said tissue, and is based at least in part on said patient data 236. Further, system 250 is configured to provide output data, which comprises the instruction data 232. Said instruction data 232, together with and/or overlaid onto said microscope image 220, can then be presented to a user, like said surgeon 210 performing the surgery on the patient 212, on a display 270. Note, that this allows also other persons present during said surgery watching the instruction data.

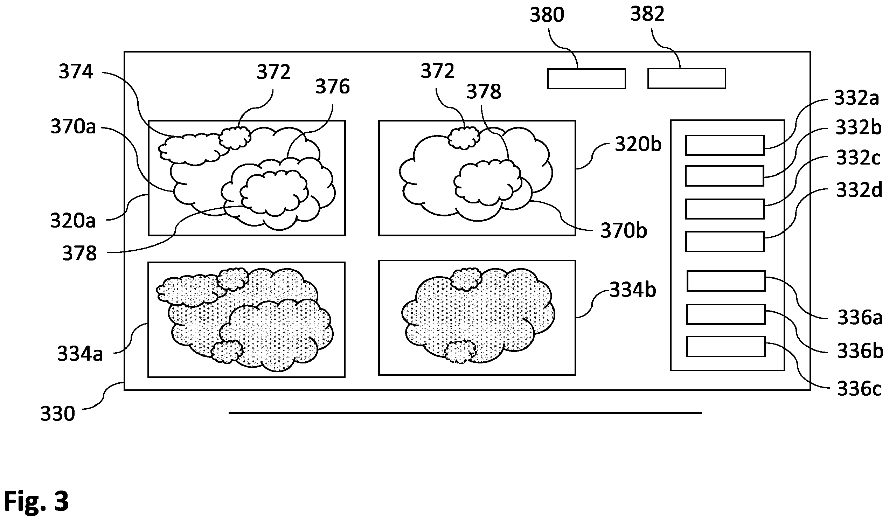

Fig. 3 schematically illustrates a way of how to create annotations for use in training of a machine-learning algorithm, which training was explained with respect to Fig. 1, for example. Within an annotation application 330, which can correspond to annotation application 130 of Fig. 1, for example, a microscope image 320a and a microscope image 320b are shown, e.g., on a display. While both, microscope image 320a and 320b, are obtained from a surgical microscope as explained with respect to Fig. 1, microscope image 320a is obtained prior to resection (or surgery), and microscope image 320b is obtained after resection (or surgery). In addition, a radiology image or scan 334a from prior to surgery and a radiology image or scan 334b from after surgery are shown. Such radiology mages or scans can be used to improve the ground truth.

Both, microscope images 320a and 320b, are, preferably, acquired with the same imaging settings; microscope image 320b can, for example, automatically be modified with respect to settings when the image is acquired, if the settings were changed while resection of tissue has taken place, to get the image of the same field of view (FoV) like for the image taken prior to resection.

As mentioned before, tissue like a brain might comprise harmful tissue like tumour that should be resected during a surgery. However, such tissue might also comprise harmful tissue like tumour that should not be resected because this tumour, for example, is located near or in a brain area in charge of some cognitive process such as speech. In this case, resecting said tumour might result in hurting said brain area such that the patient loses his or her ability of speaking. Depending on the specific type of tumour or area near which the tumour or other harmful tissue is located, the decision of whether to resect or not to resect might also be influenced by the patient data.

Such determination of actions to be performed on tissue is illustrated in Fig. 3 with the microscope image 320a prior to resection and the microscope image 320b after resection. Microscope image 320a shows tissue 370a (e.g., a brain or part thereof) prior to resection, having sections 372, 374, 376 and 378 therein. Section 378 shall be part of, the core of, tumour corresponding to section 376.

In microscope image 320b, showing tissue 370b, which corresponds to tissue 370a but after resection, only sections 372 and 378 are visible; they were not resected during surgery. Sections 374 and 376 are not visible because they were resected during surgery.

In the following, it will be described how a surgeon or other competent user or person can create annotations to be used as training data. A pair of microscope images 320a, 320b (prior to and after resection) are loaded into the annotation application 330, e.g., from database 122 shown in Fig. 1. In addition, radiology images or scans 334a, 334b (prior to and after surgery) or radiology images obtained from radiology scans are, preferably, also loaded into the annotation application 330. In addition, ultrasound images, endoscope images, and/or neuromonitoring information might also be loaded into the annotation application 330.

The microscope images 320a, 320b and the radiology images (or scans) 334a, 334b shall be aligned to each other in order to receive images (scans) having the same field of view, such they can be compared to each other.

Further, patient data like age 336a and blood pressure 336b (e.g., as a mean value or the like) of the patient from which the microscope images 320a, 320b are acquired, are provided in the annotation application 330. Patient data also includes, in particular, a remaining life time 336c of the respective patient after the resection of tissue having taken place, as well as parameters that "measure" patient's life quality after surgery such as tumour recurrence and loss of some cognitive functions in the form of e.g. categorical variables. Patient data, preferably, also include measures of oncological treatment (e.g. dosage of radio- and/or chemo-therapy).

Then, said surgeon can annotate on the microscope images based on sections visible prior to and after resection and based on the surgeon's knowledge and based on the patient data. It is noted that such annotation can also be performed based on the microscope image 320a prior to resection only, because it shows all harmful tissue ever present. Nevertheless, the microscope image 320b after the resection can, in addition be used.

In the case shown in Fig. 3, section 374 is not present in microscope image 320b, because it has been resected. The surgeon can decide that section 374 was tumour and that is was resected correctly. The surgeon can create annotation 332a including the information that section 374 is tumour and is (clearly) to be resected, for example. Section 376 is not present in microscope image 320b, it has been resected. The surgeon can decide that section 376 was tumour and that is was resected correctly. The surgeon can create annotation 332b including the information that section 376 is tumour and is (clearly) to be resected, for example.

Section 372 is also present in microscope image 320b. The surgeon can decide that section 372 was (or is) tumour but was not resected for a specific reason. For example, this might have been due to the patient's age. The surgeon can create annotation 332c including the information that section 372 is tumour but is not to be resected, for example. Also, such annotation 332c can include the information that section 372 is tumour but is not to be resected for the specific age the patient has, for example.

Section 378, the core of tumour / section 376 is also present in microscope image 320b. The surgeon can decide that section 378 was (or is) tumour but was not resected for a specific reason. For example, this might have been due to the patient's age. The surgeon can create annotation 332d including the information that section 378 is tumour but is not to be resected, for example. Also, such annotation 332d can include the information that section 378 is tumour but is not to be resected for the specific age the patient has, for example.

Additional information like tissue categories or classes 380, e.g., HGG (high grade glioma) core, HGG high density margin, HGG low density margin, LGG (low grade glioma), or healthy, and anatomical structures 382, e.g., arteries, veins, which are shown in the microscope images 320a, 320b can be displayed to support the surgeon and be annotated to support the training of the machine-learning algorithm.

For example, depending on the category or class 380 of the harmful tissue, e.g., section 378, the surgeon can create annotation 332d including the information that section 378 is tumour and is to be resected or not. Also, the remaining life time 336c can be considered as will be explained later.

Even with complete delineation of tumour tissues (core and margins, like sections 378 and 376) there is still a difficulty in deciding where to set a resection boundary, in order to maximize life expectancy without compromising life quality. Note that both, postoperative life expectancy and quality, depend on other health parameters (e.g. blood pressure, cholesterol etc.) and illnesses of patient as mentioned above (patient data). All these parameters (tumour type and size, age, blood pressure, cholesterol, various illnesses etc.) might be taken into account when trying to maximize life expectancy without compromising life quality by maximizing what to resect during surgery.

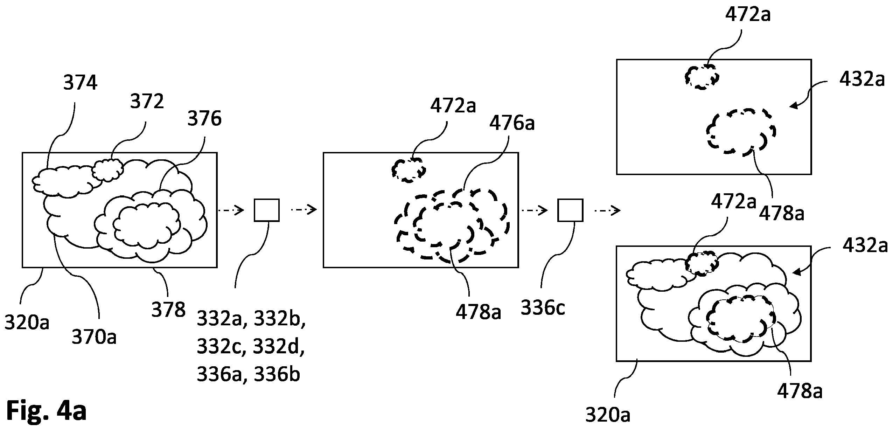

Fig. 4a schematically illustrates instruction data generated by application of a machine-learning algorithm. On the left side, Fig. 4a shows microscope image 320a from prior to resection, as shown in Fig. 3. Based on the annotations 332a, 332b, 332c, 332d that were created as explained with respect to Fig. 3, and also based on the patient data 336a, 336b, for example, instruction data 432a can be created or generated. Two different ways of how to create or present such instruction data 432a are illustrated on the right side of Fig. 4a, as will be explained below. Such instruction data 432a can comprise boundaries like boundaries 472a, 478a.

The instruction data 432a is indicative of an action to be performed on tissue; for example, said instruction data 432a can include the information that a specific section of said tissue is to be resected, for example, the tissue within boundaries 472a and 478a shall be resected. This can be provided for every part of tissue 370a.

As mentioned, such instruction data 432a can be provided or generated in the form of or comprising a boundary or resection boundary or several of such boundaries. In an embodiment, such boundary 478a, for example, is determined out of multiple boundaries 476a, 478a with different values for a parameter relating to said tissue or a patient from which said tissue is from, such that said value for said parameter is maximised, in particular, if the regression method is used. Such intermediate step is illustrated in the middle of Fig. 4a. Boundary 478a corresponds to section 378, which is the core of a tumor, for example. Boundary 476a corresponds to section 376, which is the end of margin or end of the diffusive part of the same tumor, for example. Depending on the patient data, for example, both boundaries, 476a, 478a, might be considered as for defining tissue to be resected. However, if tissue within boundary 476a (which includes tissue within boundary 478a) was resected, this might lead to less remaining life time and/or less life quality of the patient, as if only tissue within boundary 478a would be resected. Thus, by maximizing reaming life time 336c with a machine learning algorithm trained such that it considers life quality by having categorical variables such as tumor recurrence and loss of some cognitive functions, for example, boundary 478a is chosen to be included in the instruction data 432a. Note that, for example, such boundary indicating the area to be resected might also encircle the area between the lines 476a and 478a.

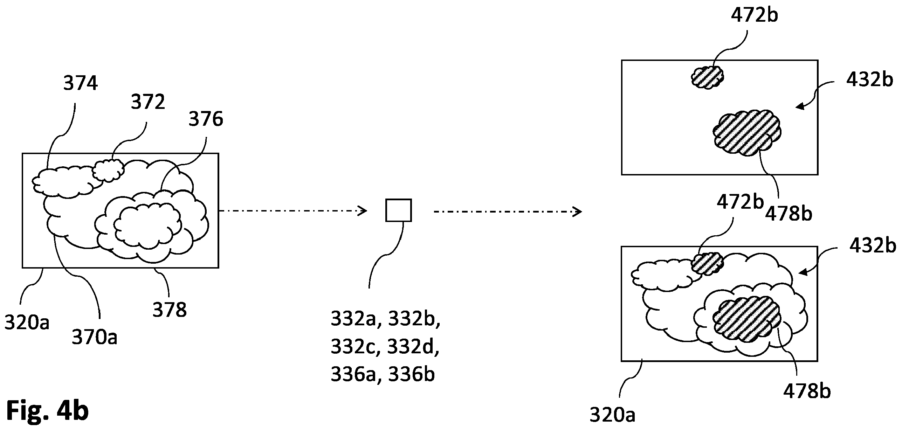

Fig. 4b schematically illustrates instruction data generated by application of a machine-learning algorithm. On the left side, Fig. 4b shows microscope image 320a from prior to resection, as shown in Fig. 3. Based on the annotations 332a, 332b, 332c, 332d that were created as explained with respect to Fig. 3, and also based on the patient data 336a, 336b, for example, instruction data 432b can be created or generated. Two different ways of how to create or present such instruction data 432b are illustrated on the right side of Fig. 4b, as will be explained below. Such instruction data 432b can comprise indicators like colors or shadings. In an embodiment, such indicator, for example, is determined based on the features of pixels in said image 320a.

Area with indicator (shading) 478b corresponds to section 378, which is classified as a core of a tumor, for example, and it is to be resected. Similarly, area with indicator (shading) 472a might have been classified as tumor and is to be resected.

Such instruction data 432a, 432b in the form of a boundary or in the form of indicators (shaded area) can be presented to a surgeon during a surgery in order to assist the surgeon in his or her decision of whether or not to resect specific parts of the tissue. It is noted that the type of lines indicating the boundaries is just an example. Any other appropriate way to indicated such area, which is to be resected could be chosen. For example, the area within said boundary, which is to be resected, could be shaded; this might be particularly useful, if the area to be resected is ring-shaped or the like.

As mentioned, different ways of how to present or generate said instruction data 432a, 432b are possible. On top of the right side of each of Figs. 4a and 4b, only the instruction data 432a, 432b in form of two boundaries or areas 472, 478 are shown. This might be displayed next to original microscope image 320a, for example. On bottom of the right side of each of Figs. 4a and 4b, the instruction data 432a, 432b in form of two boundaries 472a, 472a or indicators 472b, 478b is shown being overlaid onto original microscope image 320a. This allows the surgeon, for example, to see what parts of tissue to resect.

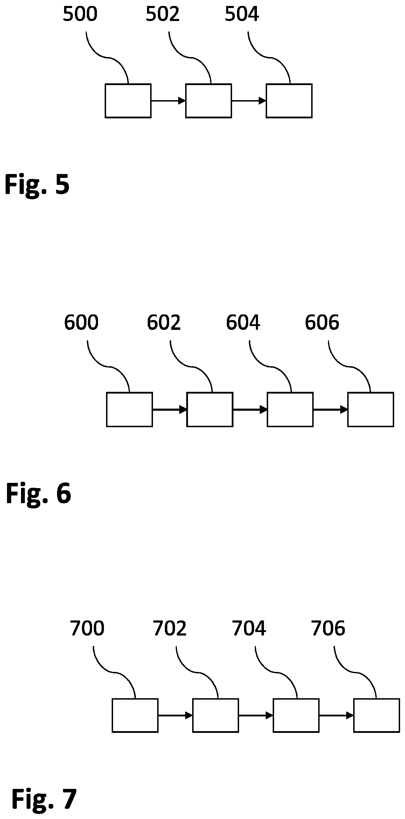

Fig. 5 schematically illustrates a computer-implemented method for training of a machine-learning algorithm according to an embodiment of the invention by means of a flow diagram. In a step 500, training data is received, which training data comprises: images showing tissue. According to an embodiment of the invention, the training data further comprises patient data, which includes information about patients correlated to said tissue shown said images; for example, parameters like age, gender, type and dosage of oncological treatment, remaining life time, blood pressure, cholesterol might be included. According to an embodiment of the invention, the training data further comprises annotations on the images, as explained with respect to Figs. 3 and 4a, 4b. Such annotations are indicative of at least one of: classes or types of tissue shown in said images, and actions to be performed or having been performed on said tissue.

In a step 502, the machine-learning algorithm is adjusted (trained), based on the training data for adjusting the machine-learning algorithm; said training is such that the machine-learning algorithm (later, when applied), generates instruction data 232for at least part of said tissue shown in said images 120, wherein said instruction data 232 is indicative of an action to be performed on said tissue; the adjusted (trained) machine-learning algorithm is then provided, in a step 504, for application.

Fig. 6 schematically illustrates a computer-implemented method for providing instruction data for tissue shown in a microscope image according to a further embodiment of the invention, by means of a flow diagram. In step 600, input data is received. The input data comprises a microscope image from a surgical microscope, obtained during a surgery, e.g., in real-time. The microscope image shows tissue of a patient. In a step 602, instruction data 232, 432 for at least part of said tissue shown in said microscope image 220 is generated, by applying a machine-learning algorithm; said instruction data is indicative of an action to be performed on said tissue. In a step 604, output data is provided, which comprises the instruction data. In a step 606, the instruction data can be displayed at a display.

Fig. 7 schematically illustrates a method for providing an image and instruction data to a user according to a further embodiment of the invention, by means of a flow diagram. In step 700, tissue of a patient is illuminated. In step 702, a microscope image of the tissue is captured by means of the surgical microscope (with image sensor); this microscope image shows the tissue. In step 704, the instruction data is generated, by applying a machine-learning algorithm, as explained with respect to Figs. 2 and 6, for example. In step 706, the microscope image and the instruction data are provided to the user of the surgical microscope, e.g., on a display.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some embodiments relate to a (surgical) microscope comprising a system as described in connection with one or more of the Figs. 1 to 7. Alternatively, a microscope may be part of or connected to a system as described in connection with one or more of the Figs. 1 to 7. Fig. 2 shows a schematic illustration of a system configured to perform a method described herein. The system comprises a microscope 200 and a computer system 250 The microscope 200 is configured to take images and is connected to the computer system 250. The computer system 250 is configured to execute at least a part of a method described herein. The computer system 250 may be configured to execute a machine learning algorithm. The computer system 250 and microscope 200 may be separate entities but can also be integrated together in one common housing. The computer system 250 may be part of a central processing system of the microscope 200 and/or the computer system 250 may be part of a subcomponent of the microscope 200, such as a sensor, an actor, a camera or an illumination unit, etc. of the microscope 200.

The computer system 150 or 250 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 150 or 250 may comprise any circuit or combination of circuits. In one embodiment, the computer system 150 or 250 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit Other types of circuits that may be included in the computer system 150 or 250 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system X20 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 250 may also include a display device, one or more speakers, and a keyboard and/or controller, which can include a mouse, trackball, touch screen, voice-recognition device, or any other device that permits a system user to input information into and receive information from the computer system 150 or 250.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

Embodiments may be based on using a machine-learning model or machine-learning algorithm. Machine learning may refer to algorithms and statistical models that computer systems may use to perform a specific task without using explicit instructions, instead relying on models and inference. For example, in machine-learning, instead of a rule-based transformation of data, a transformation of data may be used, that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output By training the machine-learning model with a large number of training images and/or training sequences (e.g. words or sentences) and associated training content information (e.g. labels or annotations), the machine-learning model "learns" to recognize the content of the images, so the content of images that are not included in the training data can be recognized using the machine-learning model. The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model. The provided data (e.g. sensor data, meta data and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e. each training sample is associated with a desired output value. By specifying both training samples and desired output values, the machine-learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g. a classification algorithm, a regression algorithm or a similarity learning algorithm. Classification algorithms may be used when the outputs are restricted to a limited set of values (categorical variables), i.e. the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine-learning model. In unsupervised learning, (only) input data might be supplied and an unsupervised learning algorithm may be used to find structure in the input data (e.g. by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e. outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component

In some examples, the machine-learning algorithm may use a decision tree as a predictive model. In other words, the machine-learning model may be based on a decision tree. In a decision tree, observations about an item (e.g. a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g. be used to store, manipulate or apply the knowledge.

Machine-learning algorithms are usually based on a machine-learning model. In other words, the term "machine-learning algorithm" may denote a set of instructions that may be used to create, train or use a machine-learning model. The term "machine-learning model" may denote a data structure and/or set of rules that represents the learned knowledge (e.g. based on the training performed by the machine-learning algorithm). In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g. of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e. to achieve a desired output for a given input

Alternatively, the machine-learning model may be a support vector machine, a random forest model or a gradient boosting model. Support vector machines (i.e. support vector networks) are supervised learning models with associated learning algorithms that may be used to analyze data (e.g. in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection.

### List of Reference Signs

- 100,200: surgical microscope
- 102, 104, 202, 204: illumination optics
- 106,206: imaging sensor
- 110,210: surgeon
- 112,212: patient
- 118,218: raw data
- 120, 220, 320a, 320b: microscope image
- 122,140: database
- 130, 330: annotation application
- 132, 332a, 332b, 332c, 332d: annotations
- 134, 334a, 334b: radiology images
- 136, 336a, 336b: patient data
- 142: training data
- 150,250: system
- 152,252: processor
- 154,254: storage device
- 160: machine-learning algorithm
- 164, 264: trained machine-learning algorithm
- 224: corrected microscope image
- 232, 432a, 432b: instruction data
- 270: display
- 370a, 370b: tissue
- 372, 374, 376, 378: sections of tissue
- 380,382: information
- 472a, 476a, 478a: boundary
- 472b, 478b: indicator
- 500-504, 600-606, 700-706: method steps

## Claims

1. A system (150) comprising one or more processors (152) and one or more storage devices (154), for training of a machine-learning algorithm (160), wherein the system (150) is configured to:
receive training data (142), the training data comprising: images (120, 320a, 320b) showing tissue;
adjust the machine-learning algorithm (160) based on the training data (142), such that the machine-learning algorithm generates instruction data (232, 432) for at least part of said tissue shown in said images (120, 320a, 320b), wherein said instruction data (232, 432a, 432b) is indicative of an action to be performed on said tissue; and
provide the trained machine learning algorithm (164).

2. The system (150) of claim 1, wherein the training data (142) further comprises: patient data (136), which includes information about patients correlated to said tissue shown said images (120).

3. The system (150) of claim 2, said patient data (136) comprising at least one of the following parameters for said patients: age, gender, type and dosage of oncological treatment, remaining life time, blood pressure, cholesterol, tumour recurrence, loss of at least part of one or more cognitive functions.

4. The system (150) of any one of claim 2 or 3, wherein the machine-learning algorithm (160) is adjusted such that the machine-learning algorithm generates said instruction data (232, 432a, 432b) such that the instruction data is based at least in part on at least parts of said patient data (136), wherein said patient data is to be provided to the trained machine-learning algorithm (164) as input data for its application.

5. The system (150) of any one of the preceding claims, the training data (142) further comprising annotations (132) on the images,
wherein the annotations are indicative of at least one of: classes or types of tissue shown in said images (120), and actions to be performed or having been performed on said tissue.

6. The system (150) of any one of the preceding claims, wherein said trained machine learning (164) algorithm is obtained based on supervised learning.

7. The system (150) of claim 5 and 6, wherein said machine learning (164) algorithm is based on classification, wherein classification is based on at least part of said annotations on the images.

8. The system (150) of claim 6 or 7, wherein said machine learning (164) algorithm is based on regression.

9. The system (150) of any one of claims 1 to 4, wherein said trained machine learning algorithm is obtained based on unsupervised learning.

10. The system (150) of any one of the preceding claims, wherein said images (120, 320a, 320b) showing tissue comprise microscope images from a surgical microscope (100) obtained during a surgery.

11. The system (150) of any one of the preceding claims, the training data (142) further comprising at least one of: radiology images (134) or scans, ultrasound images, endoscope images, neuromonitoring information.

12. The system (150) of claims 10 and 11, wherein the images showing tissue further comprise at least one of: radiology images (134), ultrasound images, and endoscope images; each showing tissue corresponding to tissue shown in the microscope images, and having the same or a similar field of view as the corresponding microscope images have.

13. The system (150) of any one of the preceding claims, wherein said instruction data (232, 432a,) comprises a boundary (472a, 478a), which is indicative of an area of said tissue, in which area said action is to be performed on said issue.

14. The system (150) of claim 13, configured to determine said boundary (478a) out of multiple boundaries (476a, 478a) with different values for a parameter relating to said tissue or a patient from which said tissue is from, such that said value for said parameter is maximised.

15. The system (150) of any one of the preceding claims, wherein said instruction data (232, 432b) comprises different indicators (472b, 478b) for different actions to be performed on said tissue.

16. The system (150) of any one of the preceding claims, wherein the machine-learning algorithm (160) is adjusted such that the machine-learning algorithm generates said instruction data (232, 432a, 432b) being overlaid to said microscope images (120, 220, 320a).

17. A computer-implemented method for training of a machine-learning algorithm (160), comprising:
Receiving (500) training data (142), the training data comprising: images (120, 320a, 320b) showing tissue;
adjusting (502) the machine-learning algorithm (160) based on the training data (142), such that the machine-learning algorithm generates instruction data (232, 432) for at least part of said tissue shown in said images (120, 320a, 320b), wherein said instruction data (232, 432) is indicative of an action to be performed on said tissue; and
providing (504) the trained machine learning algorithm (164).

18. A trained machine-learning algorithm (164), trained by:
receiving training data (142), the training data comprising: images (120, 320a, 320b) showing tissue; and
adjusting the machine learning algorithm (160) based on the training data, (142) such that the machine-learning algorithm generates instruction data (232, 432) for at least part of said tissue shown in said images (120), wherein said instruction data (232, 432) is indicative of an action to be performed on said tissue.

19. A system (250) comprising one or more processors (252) and one or more storage devices (254), for providing instruction data (232, 432a, 432b) for tissue shown in a microscope image (220), wherein the system (250) is configured to:
receive input data, the input data comprising: a microscope image (220) from a surgical microscope (200) obtained during a surgery, the microscope image (220) showing tissue of a patient,
generate instruction data (232, 432a, 432b) for at least part of said tissue shown in said microscope image (220), by applying a machine-learning algorithm (264), wherein said instruction data (232, 432a, 432b) is indicative of an action to be performed on said tissue; and
provide output data, the output data comprising: the instruction data (232) for said microscope image (220).

20. The system (250) of claim 19, the input data further comprising: patient data (236); wherein the instruction data (236) is determined based at least in part on at least parts of said patient data (236).

21. The system (250) of claim 19 or 20, the output data comprising: said microscope image (220) with said instruction data (232, 432a, 432b) being overlaid.

22. The system (250) of any one of claims 19 to 21, wherein the trained machine-learning algorithm (164) of claim 18 is used.

23. A surgical microscopy system, comprising a surgical microscope (200), an image sensor (206) and the system (250) of any one of claims 19 to 22.

24. A computer-implemented method for providing instruction data (232) for tissue shown in a microscope image (220), comprising:
receiving (600) input data, the input data comprising: a microscope image (220) from a surgical microscope (200) obtained during a surgery, the microscope image (220) showing tissue of a patient,
generating (602) instruction data (232, 432) for at least part of said tissue shown in said microscope image (220), by applying a machine-learning algorithm (264), wherein said instruction data (232, 432a, 432b) is indicative of an action to be performed on said tissue; and
providing (604) output data, the output data comprising: the instruction data (232, 432a, 432b) for said microscope image (220).

25. A method for providing an image (220) and instruction data to a user (210) using a surgical microscope (200), comprising:
illuminating (700) tissue of a patient (212),
capturing (702) a microscope image (220) of the tissue, the microscope image (220) showing the tissue,
generating (704) instruction data (232, 432a, 432b) for at least part of said tissue shown in said microscope image by applying a machine-learning algorithm (164, 264), wherein said instruction data is indicative of an action to be performed on said tissue; and
providing (706) said microscope image and said instruction data to the user (210) of the surgical microscope (200).

26. A computer program with a program code for performing the method of claim 17 or 24, when the computer program is run on a processor.
